# EUROPEAN PATENT APPLICATION

(11) **EP 4 176 934 A1**
(43) Date of publication of application: **10.05.2023**
(21) Application number: 22205680.6
(22) Date of filing: 06.11.2022
(51) Int. Cl.: A61P 3/04, A61K 38/00, C07K 14/475

(54) **LIPIDIZED COCAINE- AND AMPHETAMINE-REGULATED TRANSCRIPT PEPTIDE ANALOGUES AS ANTI-OBESITY AND NEUROPROTECTIVE AGENTS**

(30) Priority: 08.11.2021 WO PCT/CZ2021/050127
(71) Applicant: Ustav organicke chemie a biochemie AV CR, v.v.i., 166 10 Praha 6 (CZ)
(72) Inventor: Maletinska, Lenka, 16610 Praha (CZ); Zelezna, Blanka, 16610 Praha (CZ); Kunes, Jaroslav, Praha 4 (CZ); Pacesova, Andrea, 16610 Praha (CZ); Strnadova, Veronika, Roztoky (CZ)
(74) Representative: Harber IP s.r.o.

(57) **Abstract**

The invention provides analogues of the cocaine- and amphetamine-regulated transcript peptide (CARTp) which are anorexigenic compounds that lower food intake and ameliorate metabolic parameters connected with obesity, as well as improve neurodegeneration in brain upon peripheral administration. The synthesis of these lipidized peptides is described, as well as their pharmacological effects *in vitro* and *in vivo.*

## Description

### Field of Art

The new analogues of the cocaine- and amphetamine-regulated transcript peptide (CARTp) are anorexigenic compounds that lower food intake and ameliorate metabolic parameters connected with obesity, as well as improve neurodegeneration in brain upon peripheral administration. The synthesis of these lipidized peptides is described, as well as their pharmacological effects *in vitro* and *in vivo.*

### Background Art

Anorexigenic and/or anti-diabetic substances are considered as potential neuroprotective agents; neuroprotective properties were observed with several hormones implicated in maintaining energy homeostasis, such as leptin, insulin, analogues of glucagon-like peptide 1 (GLP-1), or prolactin-releasing peptide (PrRP). These hormones were shown to improve spatial memory, reduce Aβ plaques, or attenuate Tau hyper-phosphorylation (Maletinska et al. 2019, JEndocrinol 240, R47-R72). Nevertheless, to this day, there is still no effective treatment of Alzheimer's disease (AD), only drugs slowing down its progression.

CARTp, discovered in 1998 (Thim et al. 1998, Int J Biochem Cell Biol 30, 1281-1284), is a is a neuropeptide with strong anorexigenic properties abundantly expressed in hypothalamus (Kristensen et al. 1998, Nature 393, 72-76). Its importance in food intake regulation is manifested by the fact that CARTp is regulated by leptin, the key regulator of body weight (BW) and energy homeostasis (Kristensen et al. 1998, Nature 393, 72-76). CARTp has also been implicated in regulation of the hypothalamic-pituitary-adrenal axis at all levels indicating its role in stress response, in reward and reinforcement processes, and in addiction (Lau et al. 2014, Front Neurosci 8, 313; Singh et al. 2021, Peptides 140, 170534). Despite great effort, the CARTp receptor has not yet been discovered. However, *in vitro* experiments suggest that the receptor is in G-protein coupled receptor family (Yermolaieva et al. 2001, J Neurosci 21, 7474-7480); recent finding suggest that it could be GPR160 receptor (Yosten et al. 2020, J Clin Invest 130, 2587-2592). Previously, in our laboratory, specific binding of CARTp to rat pheochromocytoma PC12 cells (Maletinska et al. 2007, Eur J Pharmacol 559, 109-114) was described; PC12 cells are derived from rat adrenal glands and mimic artificial nervous system. Cells can be differentiated to neuronal phenotype by incubation with nerve growth factor (NGF). CARTp further induced activation of SAPK/JNK (Stress-activated protein kinase/c-Jun NH(2)-terminal kinase) pathway, and subsequent c-Jun protein expression, thus, confirming that CARTp can be involved in managing stress conditions (Nagelova et al. 2014, Brain Res 1547, 16-24).

Physiologically, pro-CART(1-102) is spliced into two biologically active isoforms; CARTp(55-102) and the shorter CARTp(61-102) (Thim et al. 1998, Int J Biochem Cell Biol 30, 1281-1284). Both isoforms contain three disulfide bridges between cysteines in positions 68-86, 74-94, and 88-101 which are crucial for proper biological activity (Thim et al. 1998, Int J Biochem Cell Biol 30, 1281-1284). Comprehensive study from our laboratory performed by Maixnerová et al. was focused on identifying shorter biologically active CARTp fragment able to bind to PC12 and inhibit food intake. A series of fragments corresponding to the sequences of structural loops were designed and tested. Even though some of these analogues preserved binding affinity to PC12 cells in the mM range, none of them was able to inhibit food intake in mice after intracerebroventricular (ICV) administration (Maixnerova et al. 2007, Peptides 28, 1945-1953). Following this study, the importance of individual CARTp disulfide bridges was examined (Blechova et al. 2013, Peptides 39, 138-144). A series of CARTp(61-102) variants with different combinations of preserved disulfide bridges were designed and tested. This resulted in discovery of a CARTp(61-102) analog with two disulfide bridges, between 74-94, and between 88-101, which preserves a binding affinity comparable to natural CARTp(61-102) (in the nM range) and inhibits food intake after ICV administration (Blechova et al. 2013, Peptides 39, 138-144).

Beside the hypothalamus, CARTp is also expressed in parts of the brain connected to learning and memory, such as the cortex and the hippocampus, which is one of the first area affected in AD (Koylu et al. 1998, J Comp Neurol 391, 115-132). In *in vivo* experiments, CARTp ICV administration enhanced spatial memory in rats in the Morris Water Maze (MWM) (Upadhya et al. 2011, Life Sci 88, 322-334). Potential neuroprotective properties were tested in double transgenic mouse model of AD called APP/PS1 mice (amyloid precursor protein/ presenilin 1) bearing APPswe/PSEN1dE9 mutations which are connected to familial form of AD. Interestingly, CART mRNA levels in hippocampi were reduced in APP/PS1 mice compared to wild type controls, and the immunoreactive fibers of CARTp were detected closely to Aβ plaques. The treatment of APP/PS1 mice with CARTp resulted also in enhanced memory in the MWM, decreased Aβ, and improved synaptic plasticity (Jin, et al. 2015, Sci Rep 5, 10224; Yin et al. 2017, Neurol Res 39, 885-894). Another study showed that CARTp is located in mitochondria where it acted as antioxidant agent and improved mitochondria integrity, and subsequently increased the viability of cortical neurons (Mao et al. 2007, Eur JNeurosci 26, 624-632). All these findings suggest that CARTp is a potential neuroprotective agent, but the exact mechanism of action still needs to be elucidated.

### Disclosure of the Invention

Within the framework of the invention, we have found that the attachment of fatty acid to the N-terminus of CARTp increased functionality by an order of magnitude. The effect was observed in experiments *in vitro* and *in vivo.* Statistically highly significant reduction of food intake in fasted mice after administration of lipidized CARTp analogues, was achieved for the first time even after peripheral (subcutaneous, SC) administration.

The first aspect of the invention are lipidized peptides, formed by 42 amino acids, of general formula: 
(N-x)KYGQVP-Nle ADAGEQCAVRKGARIGKLADCPRGTSCNSFLLKCL
(formula 1; SEQ ID NO: 1)
 wherein **x** is C8-C16 acyl and underlinings indicate disulfide bridges between cysteines Cys74 and Cys94 and Cys88 and Cys101 (the position numbering is in relation to CARTp(61-102)).

Preferably, x is selected from C10, C12, C14 and C16 fatty acid residue (acyl), more preferably from octanoyl (oct), myristoyl (myr), and palmitoyl (palm).

(N-x) indicates that x is bound on the N-terminus of the sequence.

The second aspect of the invention are the compounds of general formula (1), selected from the group comprising:

octKYGQVPNleADAGEQCAVRKGARIGKLADCPRGTSCNSFLLKCL (formula 2; SEQ
ID NO: 2),

myrKYGQVPNleADAGEQCAVRKGARIGKLADCPRGTSCNSFLLKCL (formula 3; SEQ
ID NO: 3),

palmKYGQVPNleADAGEQCAVRKGARIGKLADCPRGTSCNSFLLKCL (formula 4;
SEQ ID NO: 4).

The third aspect of the invention are compounds of general formula 1 as described above for use as medicaments.

The fourth aspect of the invention are lipidized peptides of general formula 1 as described above for use in the treatment of obesity and/or neurodegeneration.

The fifth aspect of the invention is a pharmaceutical composition comprising as an active ingredient a lipidized peptide of general formula 1 as described above and one or more pharmaceutically acceptable carriers, polymers, or excipients.

Finally, the invention relates to use of lipidized peptide of general formula 1 as described above for the manufacturing of medicaments for the treatment of obesity and/or neurodegeneration.

For use in human medicine, the lipidized peptides of the present invention are intended to be administered as a standard pharmaceutical composition. The present invention therefore provides a pharmaceutical composition comprising a compound of formula 1 and a pharmaceutically acceptable carrier or excipient to form appropriate formulation, microformulation or nanoformulation. Since natural CARTp with the three disulfide bridges is difficult to synthesize and the yield of the synthesis is low, the pharmaceutical composition with only two disulfide bridges is more convenient concerning the peptide synthesis and can be for use in the treatment of any of obesity and/or neurodegeneration.

Based on structural-activity studies, analogues of human (identical to rat, mouse) CARTp(61-102) were designed and lipidized at the N-terminal amino acid with a fatty acid containing C8 to C16. The methionine in the position 67 in CARTp was replaced by a more stable norleucine. It was found that 2 disulfide bridges (between Cys74 and Cys94 and Cys88 and Cys101) are necessary for maintaining biological activity while Cys68 and Cys86 could be replaced by Ala. It was also found that for maintaining the anorexic activity in mice after central administration the shorter peptides containing the key N- or C- terminal sequences as well as shorter fragments between cysteines are not sufficient and the analogues of the natural CARTp have to be used. The numbering of the positions is vis-a-vis the CARTp(61-102).

The neuropeptide CARTp and its analogues with two disulfide bridges were prepared and lipidized by fatty acid (eg. by octanoyl, myristoyl or palmitoyl) at the N-terminus of the peptide. These analogues with different chain length specifically bind to prospective receptor on PC12 cells with high affinity in *in vitro* conditions. In fasted mice they demonstrated highly significant dose-dependent reduction in food intake (P<0.001) not only after central administration but strikingly also after peripheral administration. Lipidization of neuropeptides that affect food intake and act in the brain presents a new alternative of delivering these peptides across the blood brain barrier after peripheral administration. Moreover, these lipidized analogues were able to ameliorate metabolic parameters connected with obesity and to act in brain as a neuroprotective agens. As of today, there have been no reports of lipidized CARTp analogues which lower food intake and act as neuroprotective after peripheral administration. It should be noted that lipidized analogues of CARTp evinced stronger neuroprotective effect in comparison with natural CART(61-102) even in *in vitro* models of neurodegeneration. Thus, the improved properties of lipidized CARTp analogues are not caused only by their increased blood-brain barrier penetration, but the lipidation also increases the activity of the compounds as such.

### Brief Description of the Drawings

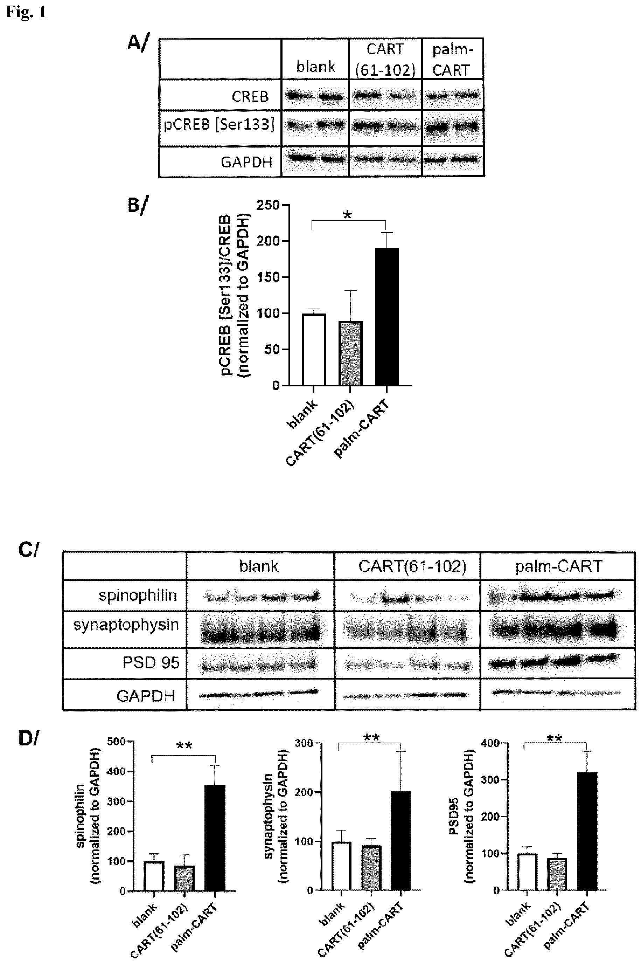

**Figure 1****:** Phosphorylation of CREB in PC12 cells represents effect of CART(61-102) and palm-CART at concentration 1×10⁻⁷ M on PC12 cells **A,B** after 10 and **C,D** 360 min-long incubation at 37 °C. Data are mean ± SEM. The significance is *P < 0.05, and **P < 0.01 versus blank (one-way ANOVA followed by Dunnett post-hoc test). 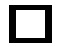 Blank 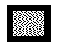 CART(61-102), ■ palm-CART.

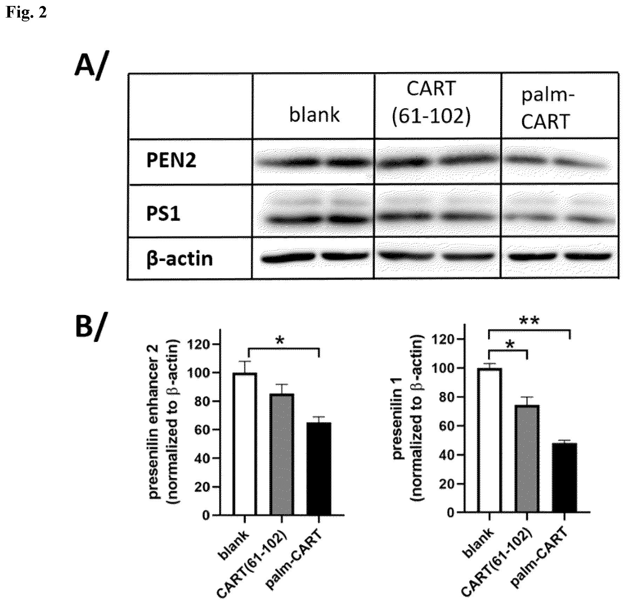

**Figure 2** represents inhibition of presenilin enhancer 2 (PEN2) and presenilin 1, both part of γ-secretase complex which cleave APP to toxic Aβ species, induced by 48-hour-long treatment with palm-CART at concentration 1×10⁻⁷ M in iPSC from patients with familial form of AD. A/ western blot, and B/ its quantification. Data are mean ± SEM. The significance is *P < 0.05, and **P < 0.01 versus blank (one-way ANOVA followed by Dunnett post-hoc test). 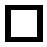 Control 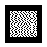 CART(61-102), ■ palm-CART.

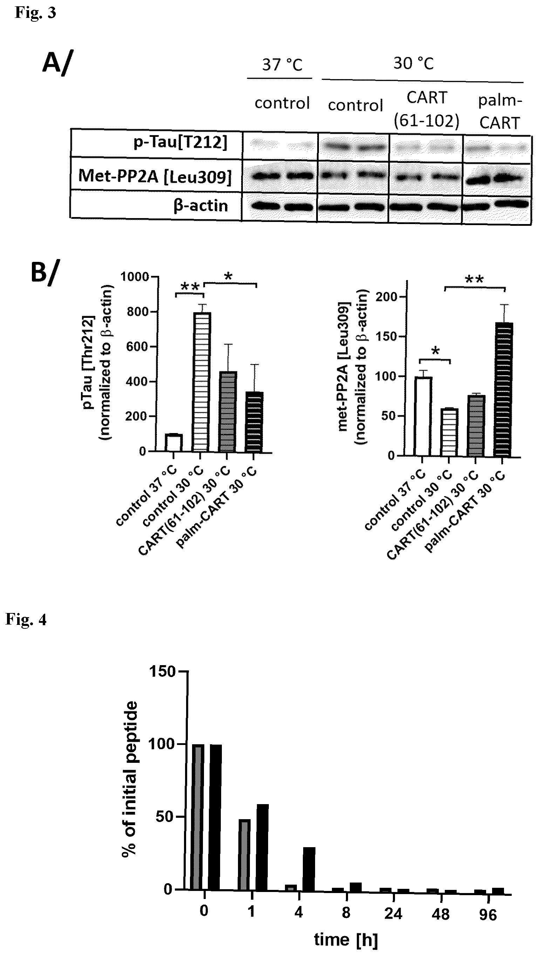

**Figure 3** represents attenuation of Tau phosphorylation at Thr212 after incubation with palm-CART at concentration 1×10⁻⁷ M in in-vitro model of Tau hyperphosphorylation induced by 2-hour-long exposure to hypothermic condition (30 °C). Attenuation of Tau hyperphosphorylation is related to increased activation of PP2A manifested by its increased methylation at Leu309. A/ western blot, and B/ its quantification. Data are mean ± SEM. The significance is *P < 0.05, and **P < 0. (one-way ANOVA followed by Dunnett post-hoc test). 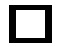 Control 37 °C, control 30 °C, CART(61-102) 30°C, 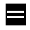 palm-CART 30°C.
Figure 4 represents the time course of stability of CARTp analogues in rat plasma measured by ELISA. CART 2S-S or palm-CART dissolved in aqueous 0.5% formic acid were added into the conditioned EDTA plasma at concentration 100 ng.ml⁻¹ and incubate at 37 °C for 96 hours; 50 µl of sample in duplicates were taken in several time points. On the x axis is time in hours, on the y axis is % of initial added peptide. 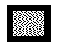 CART 2S-S, ■ palm-CART

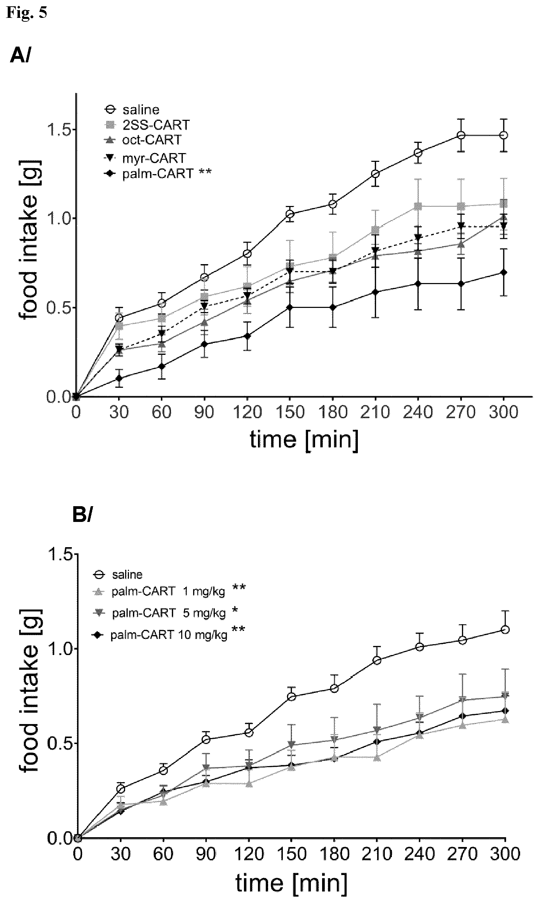

**Figure 5A** represents time course of food intake after SC administration of CARTp analogues with varying length of the lipid chain to fasted C57BL/6 mice at a dose of 10 mg/kg and 5B food intake after palm-CART at a dose 1, 5 or 10 mg/kg. On the x axis is time in minutes, on the y axis is the cumulative food intake in grams (n = 6 mice per group). The significance is *P < 0.05, **P < 0.01, and ***P < 0.001 versus saline treated group (one-way ANOVA followed by Bonferroni post-hoc test). 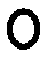 saline, 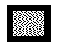 CART 2S-S, 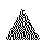 oct-CART, 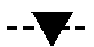 myr-CART, ◆palm-CART 10 mg/kg, 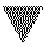 palm-CART 5 mg/kg, and 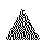 palm-CART 1 mg/kg.

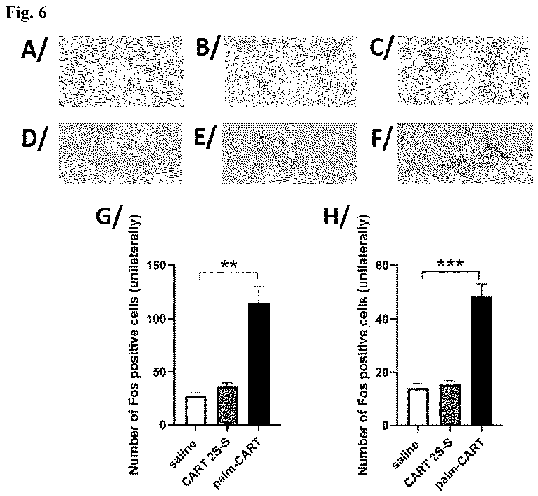

**Figure 6** represents the neuronal activation in PVN (A, B, C, G) and Arc (D, E, F, H) after SC administration of saline (A, D), CART 2S-S (B, E) and palm-CART (C, F) manifested by c-Fos. Brain slices were IHC stained using c-Fos antibody, using the staining with avidin biotin complex stained with DAB. On the y axis is the number of c-Fos positive cells (n = 3 mice per group, 5-6 slices per mice). The significance is **P < 0.01, and ***P < 0.001 versus saline treated group (one-way ANOVA followed by Dunnett post-hoc test). 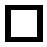 Saline, 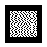 CART 2S-S, ■ palm-CART.

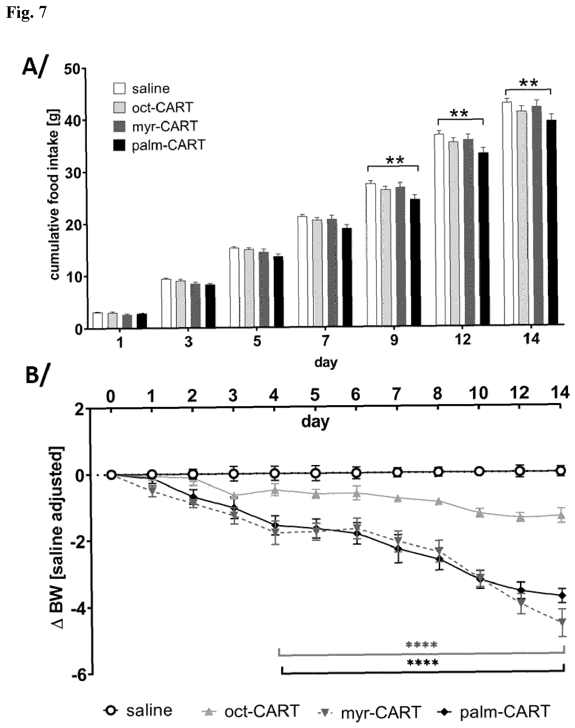

**Figure 7** represents time course of A/ cumulative food intake and B/ body weight change of DIO mice 14 days SC treated with CARTp analogues with varying length of the lipid chain at a dose 5 mg/kg once daily (15:00). On the x axes is time in days, and on the y axes is in the picture A cumulative food intake in grams and in the B ΔBW change adjusted to saline treated group (n = 10 mice per group). The significance is *P < 0.05, **P < 0.01, and ***P < 0.001 versus saline treated group (two-way ANOVA followed by Bonferroni post-hoc test). O Saline, 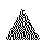 oct-CART, 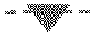 myr-CART, and ◆ palm-CART.

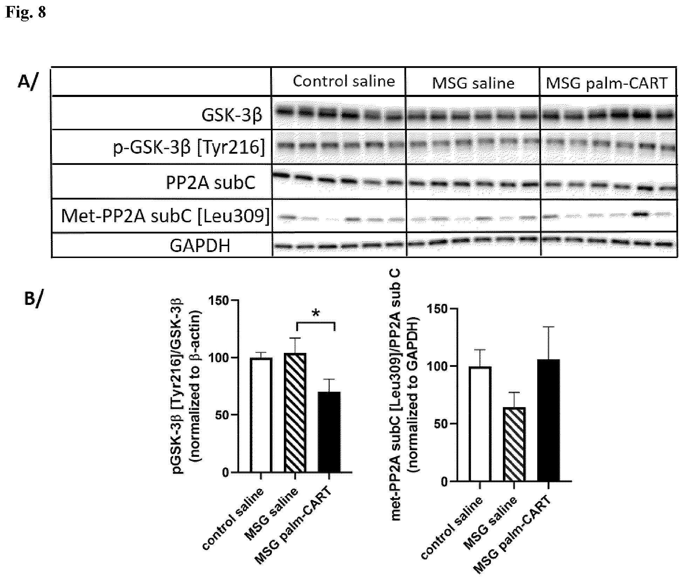

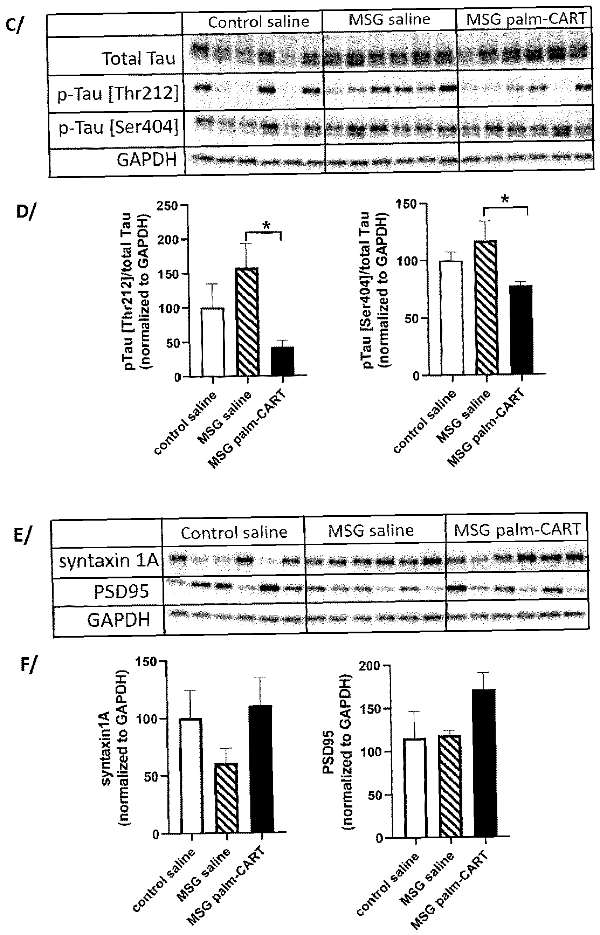

**Figure 8** represents the potential neuroprotective properties of palm-CART in hippocampi of 6-month-old MSG mice after 21 days SC treated with palm-CART. Hippocampal lysates were analyzed by the method of WB using the specific antibodies mentioned in Table 3. A/WB of GSK-3β and PP2A and B/ their quantification, C/ WB of Tau protein and D/ their quantification, E/ WB of markers of synaptogenesis and F/ their quantification. On the y axis is the level of appropriate protein normalized to GAPDH, as % of saline treated controls (n = 6 mice per group). The significance is *P < 0.05 (one-way ANOVA followed by Dunnett post-hoc test). 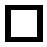 Control saline, 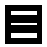 MSG saline, 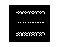 MSG palm-CART.

### List of Abbreviations

- Aβ: amyloid-β
- AD: Alzheimer's disease
- ANOVA: analysis of variance
- Arc: nucleus arcuatus
- APP: amyloid precursor protein
- BW: body weight
- CART: cocaine- and amphetamine-regulated transcript
- CREB: cAMP-response element binding
- DAB: 3,3'-diaminobenzidine
- DIO: diet-induced obesity
- EDTA: ethylenediamine tetraacetic acid
- ELISA: enzyme-linked immunosorbent assay
- GAPDH: glyceraldehyde 3-phosphate dehydrogenase
- GLP-1: glucagon-like peptide 1
- GSK-3: glycogen synthase kinase
- HF: high fat
- ICV: intracerebroventricular
- IHC: immunohistochemistry
- IL: interleukin
- iPSC: induced pluripotent stem cells
- MSG: monosodium glutamate
- MWM: Morris water maze
- Myr: myristoyl
- Oct: octanoyl
- PB: phosphate buffer
- Palm: palmitoyl
- PEN2: presenilin enhancer 2
- PP2A: protein phosphatase 2A
- PrRP: prolactin-releasing peptide
- PS1: presenilin 1
- PSD95: postsynaptic density protein
- PVN: paraventricular nucleus
- QUICKI: quantitative insulin-sensitivity check index
- SC: subcutaneous
- SEM: standard error of measurement
- SDS: sodium dodecyl sulfate
- T2DM: type 2 diabetes mellitus
- TNFα: tumor necrosis factor α
- TBS: tris-buffered saline
- WB: western blot

### Examples

### Methods of synthesis of CARTp analogues

The peptides were synthesized using the method of solid phase synthesis according to Blechová et al. (Blechova et al. 2013, Peptides 39, 138-144) utilizing the Fmoc strategy on the Liberty Blue synthesizer (CEM, CEM, Mathews, NC, USA) by stepwise coupling of the corresponding Fmoc-aminoacids to the growing chain on Fmoc-Leu-Wang LL resin. Oxyma/DIC in DMF was used as a coupling reagent. The fully protected peptide was synthesized and cleaved from the resins by a mixture of TFA/water/TIS/EDT/thioanisol (90:1:1:3:5). TFA filtrates were evaporated at room temperature by nitrogen. The residues were precipitated with tert-butyl-methylether, collected by suction, dissolved in acetonitrile/water and lyophilized. In case of lipidization with the appropriate fatty acid, DIC/HOBt was used for coupling of fatty acid to protected peptide before cleaving the peptide off the resin as previously reported (Maletinska et al. 2015, Int J Obes (Lond)).

The peptide CARTp was iodinated with Na¹²⁵I agent Iodo-Gen (Pierce, Rockford, IL, USA) according to the published procedure (Maletinska et al. 2007, Eur J Pharmacol 559, 109-114). Monoiodinated peptide was stored in aliquots at -20 °C and was used up in binding assays within one month.

**Table 1**

| **Structure of CARTp analogues** | |
|---|---|
| **Peptide** | **Sequence** |
| CART (61-102) SEQ ID NO. 5 | 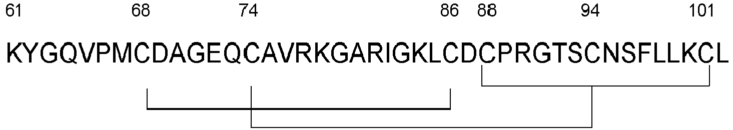 61 68 74 86 88 94 101 KYGQVPMCDAGEQCAVRKGARIGKLCDCPRGTSCNSFLLKCL |
| CART 2S-S SEQ ID NO. 6 | 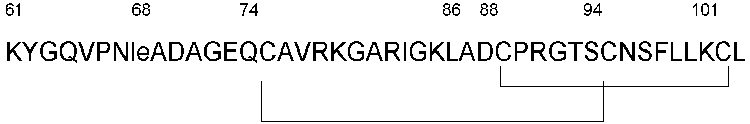 61 68 74 86 88 94 101 KYGQVPNleADAGEQCAVRKGARIGKLADCPRGTSCNSFLLKCL |
| Oct-CART SEQ ID NO. 2 | 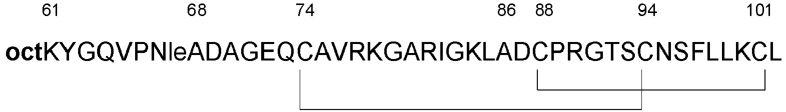 61 68 74 86 88 94 101 octKYGQVPNleADAGEQCAVRKGARIGKLADCPRGTSCNSFLLKCL |
| Myr-CART SEQ ID NO. 3 | 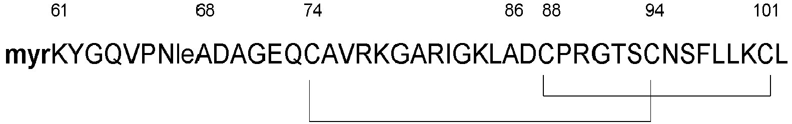 61 68 74 86 88 94 101 myrKYGQVPNleADAGEQCAVRKGARIGKLADCPRGTSCNSFLLKCL |
| Palm-CART SEQ ID NO. 4 | 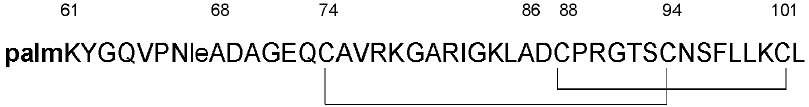 61 68 74 86 88 94 101 palmKYGQVPNleADAGEQCAVRKGARIGKLADCPRGTSCNSFLLKCL |
| Palm-CART [74-94, 86-101] SEQ ID NO. 7 | 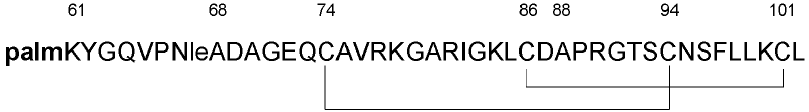 61 68 74 86 88 94 101 palmKYGQVPNleADAGEQCAVRKGARIGKLCDAPRGTSCNSFLLKCL |

### Example 1: Competitive binding experiments

The rat pheochromocytoma cell line PC12 was obtained from ATCC (Manassas, VA, USA). The cells were grown in RPMI 1640 medium (Sigma, St. Luis, MO, USA) supplemented with 10% horse serum, 5% fetal bovine serum (always v/v, unless otherwise stated), 2 mM L-glutamine (PAA Laboratories GmbH, Pasching, Austria), and 1% penicillin/streptomycin (PAA Laboratories GmbH, Pasching, Austria), and passaged once per week to maintain the cells in the exponential growth phase. For binding studies, the cells were seeded on polyethylene imine-coated 24-well plates (Corning, NY, USA). To reach a density of approximately 7 × 10⁵ cells/well, which was found to be optimal for binding experiments, the non-differentiated cells were allowed to grow for 3 days.

Competition binding experiments were performed according to Motulsky and Neubig (Motulsky et al. 2002, Curr Protoc Neurosci Chapter 7, Unit 7.5).

Plated cells were incubated with 10⁻¹⁰ M ¹²⁵I-CART(61-102) and 10⁻¹¹ - 10⁻⁶ M non-radioactive CARTp analogues in a total volume of 0.25 ml of binding buffer (20 mM HEPES buffer, pH 7.4, 118 mM NaCl, 4.7 mM KCl and 5 mM MgCl₂, 5.5 mM glucose, 1 mg/ml BSA, and 0.1 mg/ml basic pancreatic trypsin inhibitor) for 30 min at 37°C as previously described (Maletinska et al. 2007, Eur J Pharmacol 559, 109-114). Non-specific binding was determined using 10⁻⁶ M CART(61-102). After incubation, the cells were washed with washing buffer (10 mM HEPES buffer, pH 7.4, 118 mM NaCl, 4.7 mM KCl and 5 mM MgCh) and then solubilized in 0.1 N NaOH. Bound radioactivity was determined by γ-counting (Wizard 1470 Automatic Gamma Counter, Perkin Elmer, Wellesley, MA, USA). The total binding amounted to 3 - 8 % of the radioactivity added, and the non-specific binding was less than 15 % of the total binding. Experiments were carried out in duplicate at least three times. All compounds tested were dissolved in Milli-Q water (purified using an ion exchange cartridge in Merck Millipore system) and stored frozen at-20 °C in aliquots until use.

All the tested CARTp analogues (for structure, see Table 1), that is the lipidized CART peptides, were bound with high affinity to the binding site in PC12 cells (Kᵢ in the order of 10⁻⁶ - 10⁻⁹ M). With the lengthening chain of the fatty acid the value of Kᵢ was decreasing, meaning the binding was stronger, up to an order of magnitude as compared to the natural CART(61-102) (see Table 2).

**Table 2**

| **The affinity of lipidized analogues of CARTp to binding site in PC12 cells.** | | | |
|---|---|---|---|
| Displacement of ¹²⁵I-CART(61-102) by lipidized analogues of CARTp. | | | |
| Analog | Kᵢ (nM) | ¹²⁵I-CART(61-102) | % binding CART(61-102) |
| **CART(61-102)** | 9.33 ± 4.37 | | 100 |
| **CART 2S-S** | 261.30 ± 209.80 | | 3.5 |
| **Oct-CART** | 76.09 ± 17.66 | | 12 |
| **Myr-CART** | 12.10 ± 3.40 | | 78 |
| **Palm-CART** | 1.43 ± 0.41 | | 652 |
| **Palm-CART [74-94, 86-101]** | 1091.91 ± 693.76 | | 0.85 |

| | | | |
|---|---|---|---|
| Data are mean ± SEM, n = 3 experiments in duplicates. | | | |

### Evaluation of in vitro experiments.

For evaluation of competitive binding experiments, the program Graph Pad Prism Software (San Diego, CA, USA) was used.

For the competitive binding experiments, a method of nonlinear regression assuming one-site binding was used. The value of *Kᵢ* was calculated using the equation of Cheng and Prussof (Cheng et al. 1973, Biochem Pharmacol 22, 3099-3108), using the value of *K_{d}* 1.49 nM and concentration of radioligand was 0.1 nM. The importance of the disulfide bridges 74-94 and 88-101 for proper binding of the palm-CART was confirmed, since the palm-CART analog with disulfide bridges 74-94 and 86-101 shown three order of magnitude lower ability to bind to PC12 cells than CART(61-102).

### Example 2: Cellular signaling and synaptic plasticity in PC12 cells after incubation with CART(61-102) or palm-CART in PC12 cells

Since palm-CART showed higher affinity to prospective receptor, cellular signaling in PC12 cells were further examined. For the experiment, the cells were seeded on polyethylene imine-coated 12-well plates (Corning, NY, USA). To reach a density of approximately 1.5 × 10⁶ cells/well, which was found to be optimal for the experiments, the non-differentiated cells were allowed to grow for 3 days. Before the experiment, the cells were overnight incubated in growth medium without horse and fetal bovine serum. CART(61-102) and palm-CART at concentration 1x 10⁻⁷ M were added to cells and incubated for 10 or 360 minutes in 37 °C. Afterwards, the cells were washed with cold 0.1M PBS buffer pH 7.4, solubilized in Laemmli sample buffer and stored in -20 °C. Western blot (WB) was performed as previously described by (Spolcova et al. 2015, J Alzheimers Dis 45, 823-835).

CART(61-102) did not increase phosphorylation of the cAMP- response element binding (CREB) at Ser133, however, palm-CART significantly increased the phosphorylation compared to cells treated with the vehicle (blank), as shown in Fig 1 A,B. Compared to cells incubated with vehicle, palm-CART after 360 min incubation with PC12 cells significantly increased the level of several markers of synaptic plasticity, such as presynaptic proteins synaptophysin and spinophilin, and post-synaptic density protein 95 (PSD95) (Fig 1 C, D).

### Example 3: Neuroprotective properties of palm-CART in in-vitro models of neurodegeneration

As shown in Example 2, palm-CART increased the markers of synaptic plasticity. Thus, its potential neuroprotective properties were studied in in-vitro models of neurodegeneration.

First, induced pluripotent stem cells (iPSC) obtained from patient with familial form of AD were seeded on 6-well plates (Corning, NY, USA) and grown for 2 weeks in complete medium containing DMEM/F12, 0.5% N-2 supplement, 1% B27 supplement without vitamin A, 1% L-glutamine, 1% non-essential amino acids, 1% penicillin/streptomycin, and 20 ng/ml FGF2 (all from Thermo Fisher Scientific, Waltham, MA, USA). Subsequently, the iPSC were 48 hours treated with CART(61-102) or palm-CART at concentration 1x 10⁻⁷ M. Afterwards, the cells were washed with cold 0.1M PBS buffer pH 7.4, solubilized in Laemmli sample buffer and stored in -20 °C. Western blot (WB) was performed as previously described by (Spolcova, et al. 2015, J Alzheimers Dis 45, 823-835).

The treatment with palm-CART, but not CART(61-102), resulted in significantly reduced amount of presenilin enhancer 2 (PEN2) and presenilin 1 (PS1) (Fig. 2A, B), both components of γ-secretase complex responsible for cleavage of APP to toxic Aβ species.

The effect of CART(61-102) and its analogue palm-CART was further tested in model of hypothermia-induced Tau hyperphosphorylation using hippocampal cell line HT22 (Merck, Darmstadt, Germany). The day before the experiment, HT22 cells were seeded on 12-well plates (Corning, NY, USA) and grown in complete medium containing DMEM, 10% IFBS, 1% L-glutamine, and 1% penicillin/streptomycin. For 2 hours, HT22 cells were exposed to hypothermic condition (30 °C) and simultaneously treated with CART(61-102) or palm-CART at concentration 1 × 10⁻⁷ M; control cells were maintained in 37 °C. Afterwards, the cells were washed with cold 0.1M PBS buffer pH 7.4, solubilized in Laemmli sample buffer and stored in -20 °C. Western blot (WB) was performed as previously described by (Spolcova, et al. 2015, J Alzheimers Dis 45, 823-835).

Compared to control cells, HT22 cells in hypothermic condition showed significantly increased Tau hyperphosphorylation at Thr212 The phosphorylation was attenuated in cells treated with palm-CART, not with CART(61-102). Decreased phosphorylation of Tau protein at Thr212 was linked to increased activity of protein phosphatase 2A (PP2A), manifested by increased methylation at Leu309 in HT22 cells treated with palm-CART (Fig. 3A, B).

### Example 4: Stability of palmitoylated CARTp in rat plasma

Blank rat pooled EDTA plasma was conditioned in an incubator at 37 °C. Plasma was preheated to reach required temperature and then it was spiked with a solution of CART 2S-S and palm-CART analogues (dissolved in aqueous 0.5% formic acid) to a final concentration 100 ng.ml⁻¹. Duplicates were taken for ELISA analysis, sampling volume was 50 µL. Samples were immediately transferred to -20 °C.

ELISA kit for CARTp (MyBioSource, San Diego, CA, USA) was used in accordance with manufacturer's directions. In addition, external calibration using palm-CART was used. The rat plasma was applied for the calibration to comprise specific influence of different matrices on the immunoanalysis output.

Fig. 2 shows stability of nonlipidized CART 2S-S and palm-CART. Palm-CART revealed higher stability in rat plasma compared to CART 2S-S.

### Example 5: Test of food intake after SC administration of lipidized CARTp analogues

All experiments followed the ethical guidelines for animal experiments and Czech Republic Law No. 246/1992 and were approved by the committee for experiments with laboratory animals of the Czech Academy of Sciences (CAS).

Inbred C57BL/6 male mice (AnLab, Prague, Czech Republic) were housed at a temperature of 23°C under a daily cycle of 12 hours of light and dark (light from 6:00 a.m.) with free access to water and a standard chow diet that contained 25%, 9% and 66% calories from protein, fat and carbohydrate, respectively. The energy content of the diet was 3.4 kcal/g (Ssniff, Spezialdiäten GmbH, Soest, Germany).

The food was withdrawn 16 hours prior to injection of peptides, free access to water was maintained. The administration of saline, CART 2S-S or lipidized analogues was performed either by SC injection at doses of 1-10 mg/kg (volume 0.15 ml/mouse). Sixty minutes after the peptide administration, the mice were given pre-weighed food. The food was then weighed every 30 minutes for 5 hours. The administration of every dose was performed at least twice and one group of mice consisted of at least 5 mice. The results were presented in grams of food intake and compared with the control group injected with saline.

Statistical evaluation was performed using program Graph-Pad Prism Software (San Diego, CA, USA), and the one-way ANOVA with a subsequent Dunnett post-hoc test. The difference in food intake between mice injected with saline and mice injected with the examined peptide were considered statistically significant at P < 0.05.

All three lipidized CART analogues were able to decrease significantly food intake in fasted mice unlike non lipidized CART 2S-S peptide, see Fig. 5. The anorexigenic effect of lipidized CART analogues was long lasting. The most significant decrease of food intake was observed after injection of palm-CART and the effect of this analog was significant even at a dose of 1 mg/kg (Fig. 5B).

### Example 6: Fos Immunohistochemistry

### Tissue processing

For Fos immunohistochemical processing, overnight fasted mice (n=3 mice/group) were treated SC with saline, CART 2S-S, palm-CART (dose 10 mg/kg). Ninety minutes after SC injection, the mice were deeply anesthetized with pentobarbital (50 mg/kg, i.p.) and perfused transcardially with ice-cold saline supplemented with heparin (10 U/ml, Zentiva, Prague, Czech Republic). Then the brains were removed, postfixed in 4% paraformaldehyde dissolved in phosphate buffer (PB) pH 7.4 overnight at 4 °C, and infiltrated with 30% sucrose in 0.1 M PB at 4 °C until the sectioning. For sectioning, the frozen brains were placed into a Leica cryostat device adjusted to -25 °C and 30 µm coronal sections were cut from the brains and collected as free floating in cold (4°C) cryoprotective solution (25% glycerin, and 30% ethylene glycol in PB).

Free floating sections were repeatedly washed in cold Tris-buffered saline (TBS), and boiling in 85°C for 30 min in citrate buffer pH 6.0 (Abcam, Cambridge, UK) for antigen retrieval. Afterwards, slices were incubated for 30 min in 0.6% H₂O₂ for 40 min at room temperature. After 1-hour blocking in 5% goat serum dissolved in TBS with 0.2% Triton X-100 (TBS-T, Sigma-Aldrich, St. Louis, MO, USA), slices were 48 hours incubated with polyclonal Fos protein antiserum (1:500), diluted in 1% goat serum in TBS-T. After several rinses in TBS-T, the sections were incubated with biotinylated goat-anti-rabbit IgG (1:500, VectorStain Elite ABC, Vector Lab., Burlingame, CA, USA) for 90 min at room temperature. Next TBS-T rinses were followed by incubation with the avidin-biotin peroxidase complex reagent (Vectastain ABC Kit, Vector Laboratories, Burlingame, CA, USA). The samples were stained in 3,3'-diaminobenzidine solution (DAB, Vector Laboratories, Burlingame, CA, USA), mounted on silanized slides and coverslipped using VectaMount AQ mounting medium (Vector Laboratories, Burlingame, CA, USA). Stained sections were viewed and imaged under an BX53 microscope equipped with DP74 camera (Olympus, Tokyo, Japan).

### Evaluation of the immunostaining

An identical set of mice was used for determination of Fos immunoreactivity in nucleus arcuatus (Arc), and paraventricular nucleus (PVN) according to the mouse brain atlas (Franklin et al. 2008, The Mouse Brain in Stereotaxic Coordinates. Academic Press). Representative sections were captured by the same computerized system. The percentage of the stained area or the particle (stained cells) counts were then analyzed using ImageJ software (NIH, Bethesda, MD, USA).

Neural activity presented in c-Fos immunoreactivity (Fig. 6) was significantly increased in a case of palm-CART analog in brain nuclei involved in food intake regulation tested (i.e. Arc, PVN). This analog also significantly decreased food intake.

### Example 7: 14-day administration of lipopeptides in model of diet-induced (DIO mice)

Inbred C57BL/6 male mice (Charles River, Germany) were housed at a temperature of 23°C under a daily cycle of 12 hours light and dark (light from 6:00 a.m.) with free access to water and food pellets (Ssniff Spezialdiäten GmbH, Soest, Germany).

At 8 weeks of age, mice were switched to a high fat (HF) diet (the energy content of the HF diet was 5.3 kcal/g, containing 13, 60, and 27 % of calories from protein, fat, and carbohydrate, respectively). The HF diet consisted of 40 % standard St-1 diet, 34 % powdered cow milk for human neonates, 25 % lard, and 1 % corn starch w/w (Kopecky et al. 1996, Am J Physiol 270, E768-775). Food intake and BW were monitored weekly from 9 to 24 weeks of age. Mice resistant to the HF diet were withdrawn from the experiment (approximately 10 % of mice). At the age of 23 weeks, mice were placed into separate cages with free access to food and water. The following week, mice were subjected to a 14-day food intake experiment. They were injected with saline, oct-CART, myr-CART and palm-CART dissolved in saline at doses of 5 mg/kg (n=10) once daily (at 15:00) for 14 days. Consumption of HF diet and the BW of the mice were simultaneously followed.

After 14 days of treatment, mice were sacrificed in the morning. Their blood plasmas were isolated, and the white adipose tissue (subcutaneous, abdominal, and gonadal), and the liver of all mice were dissected, weighed, and stored at -80° C.

### Statistical analysis

The data of food intake and BW change are presented as means ± SEM. They were analyzed by a two-way ANOVA followed by a Bonferroni *post-hoc* test using Graph-Pad Software (San Diego, CA, USA). P < 0.05 was considered statistically significant.

Results of 14-days administration of myr-CART and palm-CART analogues into DIO mice are shown in Fig. 7 (body weight change and food intake). Food intake was significantly decreased compared to saline treated group after application of palm-CART to DIO mice. BW change of DIO mice was significantly decreased both after myr-CART and palm-CART treatment compared to saline treated group.

### Example 8: 21-day administration of lipopeptides in model of monosodium glutamate (MSG)-induced obesity with neurodegeneration

To examine neuroprotective properties, insulin resistant animal model was used, e.g. mouse model with obesity induced by MSG. These mice are characterized by growth hormone insufficiency, pituitary and optic nerves atrophy, and infertility (Olney 1969, Science 164, 719-721). In their brains the reduced *Arc,* enlarged third brain ventricle, and narrowed *eminentia mediana* are observed. Total number of neurons in Arc is reduced about 75 % in MSG mice compared to their controls; however, the number of neurons does not differ significantly in other brain regions (Elefteriou et al. 2003, Endocrinology 144, 3842-3847).

The imbalance between food intake and energy expenditure at MSG obese mice leads to hypophagia and an increased adipose tissue; compared to their control, MSG obese mice have even 8 times higher weight of white adipose tissue (Maletínská et al. 2006, *Regul Pept* **136,** 58-63). They have also increased leptin and glucose blood concentration, and insulin resistance (Maletínská et al. 2006, *Regul Pept* **136,** 58-63).

At six months of age, these mice have developed neurodegeneration in hippocampus as shown by decrease sensitivity of insulin receptor cascade and increased pathological Tau phosphorylation (Spolcova et al. 2015, J Alzheimers Dis 45, 823-835).

### MSG mice

Mice of strain NMRI (Harlan, Italy) were housed at the certified animal facility of IOCB CAS, Prague at 23 ± 2°C, they had free access to water and food. They were fed standard chow diet (Ssniff, Germany), which contained 66 % of calories as carbohydrates, 25 % as protein, and 9 % as fat; its energy content was 3.4 kcal/g. Daily cycle was 12/12 hours, lights on at 6:00 a.m. All animal experiments followed the ethical guidelines for animal experiments and the Czech Republic Act No. 246/1992.

For obesity, insulin resistance and neurodegeneration induction, the newborn NMRI mice were SC administered with sodium glutamic acid (Sigma, St. Louis, USA) at dose 4 mg/g of BW at postnatal days 2-5. These MSG-obese mice were fed the same standard diet as the control group. The food and BW were monitored once per week. For the study, MSG and control male mice at the age 6 months were used.

Groups of MSG mice (n = 10 animals per group) were for 21 days SC administered saline or palmitoylated analog of CARTp at a dose 10 mg/kg, dissolved in saline, once a day, 15:00. Control mice (n = 10 animals per group), NMRI and MSG, were injected with saline (the volume was always 0.15 ml/mouse).

### Tissue dissection

At the end of experiment, overnight fasted mice with ad libitum access to water were weighed, and their plasma glucose concentration was measured using Glucocard glucometer. After transcardial perfusion with heparinized saline solution, the brains were dissected on ice, and cut between hemispheres. For immunohistochemical staining the half of the brain was fixed for 24 hours in 4% paraformaldehyde and stored in 30% sucrose, afterwards. For the WB analysis, the hippocampus was dissected, and lysed in cold lysis buffer (62.5 mM Tris-HCl, pH 6.8 with 1% sodium deoxycholate, 1% Triton X-100, Complete, 50 mM NaF, 1 mM Na₃VO₄), homogenized, sonicated 10 minutes and stored at -20 °C. The blood plasma was prepared, and stored at - 80 °C.

Level of plasma insulin was measured by radioimmunoassay kit (Millipore, St. Charles, MI, USA), level of leptin by ELISA kit (Millipore, St. Charles, MI, USA) and cholesterol by colorimetric assay (Erba Lachema, Brno, Czech republic). All measurements were performed according to the manufacturer's instructions. Quantitative insulin sensitivity check index (QUICKI) was measured from fasting glucose and fasting insulin (QUICKI = 1/[(log I0) + (log G0)], where I0 is fasting insulin in µU/ml and G0 is fasting glucose in mg/dl).

WB analysis of GSK-3β as the main Tau kinase, and PP2A as the main Tau phosphatase, detection of hyperphosphorylation of Tau protein at different epitopes and markers of synaptogenesis were examined. Briefly, in homogenized hippocampi the protein level was measured using BCAkit (Pierce, Thermo Fisher Scientific, Rockfor, IL, USA), then the samples were diluted in sample buffer (62.5 mM Tris-HCl pH 6,8, 10% glycerol, 2% SDS, 0.01% bromfenol blue, 5% mercaptoethanol, 50 mM NaF and mM Na₃VO₄) to final concentration 1 µg/µl. WB method and analysis of the results were performed according (Spolcova et al. 2015, J Alzheimers Dis 45, 823-835). The list of the used antibodies and their dilution is shown in Table 3.

### Statistical analysis

Statistical analysis was calculated by one-way ANOVA, with Dunnett post-hoc test using GrapPad software (San Diego, CA, USA). Data are presented as mean ± SEM.

**Table 3**

| List of antibodies with their appropriate dilution and diluent | | |
|---|---|---|
| **Antibody** | **Company** | **Dilution** |
| Mouse monoclonal antibody against CREB | Cell Signaling Technology, Beverly, MA, USA | 1:1000 5% milk TBS/tween-20 |
| Mouse monoclonal antibody against phosphor-CREB [Ser133] | Cell Signaling Technology, Beverly, MA, USA | 1:1000 5% milk TBS/tween-20 |
| Mouse monoclonal antibody against GAPDH | Cell Signaling Technology, Beverly, MA, USA | 1:1000 5% milk TBS/tween-20 |
| Rabbit monoclonal antibody against phospho -GSK-3β [Tyr216] | Abcam, Cambridge, UK | 1:1000 5% BSA TBS/tween-20 |
| Rabbit monoclonal antibody against total GSK-3β | Cell Signaling Technology, Beverly, MA, USA | 1:1000 5% BSA TBS/tween-20 |
| Mouse monoclonal antibody against met-PP2A subC [Leu309] | Millipore, Temecula, CA, USA | 1:1000 5% BSA TBS/tween-20 |
| Rabbit monoclonal antibody against PP2A subC | Cell Signaling Technology, Beverly, MA, USA | 1:1000 5% BSA TBS/tween-20 |
| Rabbit polyclonal antibody against total PSD95 | Cell Signaling Technology, Beverly, MA, USA | 1:1000 5% BSA TBS/tween-20 |
| Rabbit polyclonal antibody against spinophilin | Cell Signaling Technology, Beverly, MA, USA | 1:1000 5% BSA TBS/tween-20 |
| Rabbit polyclonal antibody against synaptophysin | Santa Cruz Biotechnology Inc., Dallas, TX, USA | 1:5000 5% mild TBS/tween-20 |
| Rabbit monoclonal antibody against syntaxin 1A | Cell Signaling Technology, Beverly, MA, USA | 1:1000 5% BSA TBS/tween-20 |
| Rabbit polyclonal antibody against Tau [pS404] | Invitrogen Grand Island, NY, USA | 1:10 000 5% BSA TBS/tween-20 |
| Rabbit polyclonal antibody against Tau [pT212] | Invitrogen Grand Island, NY, USA | 1:1000 5% BSA TBS/tween-20 |
| Mouse monoclonal antibody against total Tau (Tau5) | Thermofisher, Rockford, IL, USA | 1:10 000 5% milk TBS/tween-20 |

Compared to control mice treated with saline, MSG saline treated mice displayed increased BW. 21-day-long SC treatment of 6-month-old MSG mice with palm-CART at a dose 10 mg/kg resulted in decreased BW compared to MSG saline treated group. Moreover, decreased level of leptin, insulin and cholesterol were observed. Subsequently, the treatment with palm-CART improved peripheral insulin sensitivity manifested by increased level of QUICKI (Table 4). In hippocampi, decreased phosphorylation of GSK-3β in hippocampi of MSG mice manifested by decreased phosphorylation at Tyr216 which displayed its decreased kinase activity towards Tau protein (Fig. 8 A, B). Moreover, increased methylation at Leu309 pointing to increased activation of phosphatase activity towards Tau protein (Fig. 6 A, B). Subsequently, attenuated Tau phosphorylation was observed in hippocampi of MSG mice treated with palm-CART at epitopes Thr212 and Ser404 (Fig. 8C, D). Treatment with palm-CART showed beneficial effect on synaptic plasticity of MSG mice, since increased level of pre-synaptic marker syntaxin 1A and post-synaptic marker PSD95 were observed (Fig. 8 E, F).

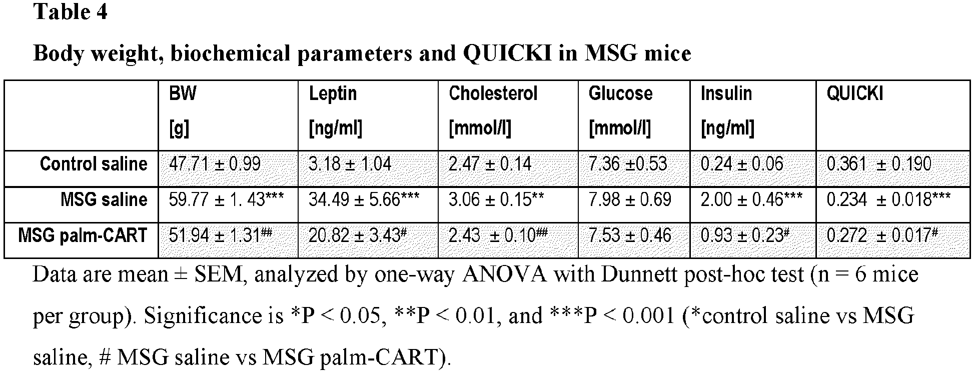

In summary, it was found that
- The lipidization of peptides, analogues of CARTp, at the N-terminus allows for the central effect of the neuropeptide which is endogenously expressed in the hypothalamus, after subcutaneous (peripheral) administration.
- The lipidization of all these peptides led to the increase in receptor binding - it lowered the Kᵢ in binding to PC 12 cells in correlation with the chain length of the fatty acid. The addition of fatty acid not only preserved receptor binding (as a result of N-terminal lipidization), it increased the binding affinity by an order of magnitude.
- All the tested peptides were agonists of CARTp (which was determined food intake effect in mice).
- In the food intake test after SC administration to mice the peptides lipidized with oct, myr and palm, significantly reduced food intake in the dose-dependent manner. This is the first ever report of a modified CARTp that reduces food intake also after peripheral administration.
- After SC administration to mice, palmitoylated CARTp analog increased neuronal activity in brain (manifested by c-Fos) unlike non-lipidized CARTp.
- The effect of lowering food intake was long-lasting, and in a dose-dependent manner persisted for more than 10 hours after administration. This is most likely due to increased resistance to degradation by proteases (introduction of fatty acid), and to the binding of lipopeptide to serum albumin.
- In the test of stability in rat plasma, palmitoylated CARTp showed higher stability compared to non-lipidized analog.
- The most effective of the tested analogues of CARTp was palmitoylated CARTp analog.
- Palmitoylated CARTp analog showed lowering of food intake and body weight after chronic treatment in mouse model of obesity.
- Palmitoylated CARTp analog showed neuroprotective effect: increased markers of synaptic plasticity and decreased phosphorylation of protein Tau as a feature of Alzheimer's like pathology.

### Industrial applicability

Potential anti-obesity and neuroprotective agents with subcutaneous administration.

## Claims

1. Lipidized cocaine- and amphetamine-regulated transcript peptide analogue of general formula 1: 
(N-x)KYGQVP-Nle ADAGEQCAVRKGARIGKLADCPRGTSCNSFLLKCL (SEQ
ID NO: 1),
 wherein **x** is an C8-16 acyl; and underlinings indicate disulfide bridges.

2. Lipidized cocaine- and amphetamine-regulated transcript peptide analogue of general formula 1 according to claim 1, wherein x is selected from C10, C12, C14 and 16 acyl.

3. Lipidized cocaine- and amphetamine-regulated transcript peptide analogue of general formula 1 according to claim 1, wherein x is selected from octanoyl, myristoyl and palmitoyl.

4. Lipidized cocaine- and amphetamine-regulated transcript peptide analogue of general formula 1 according to claim 1, selected from the group comprising: 
octKYGQVPNleADAGEQCAVRKGARIGKLADCPRGTSCNSFLLKCL (formula 2; SEQ
ID NO: 2),
 
myrKYGQVPNleADAGEQCAVRKGARIGKLADCPRGTSCNSFLLKCL (formula 3; SEQ
ID NO: 3),
 
palmKYGQVPNleADAGEQCAVRKGARIGKLADCPRGTSCNSFLLKCL (formula 4;
SEQ ID NO: 4),
 wherein oct is octanoyl, myr is myristoyl and palm is palmitoyl.

5. Lipidized cocaine- and amphetamine-regulated transcript peptide analogue according to any one of claims 1 to 4, for use as a medicament.

6. Lipidized cocaine- and amphetamine-regulated transcript peptide analogue according to any one of claims 1 to 4 for use in the treatment of obesity and/or neurodegeneration.

7. Lipidized cocaine- and amphetamine-regulated transcript peptide analogue according to any one of claims 1 to 4 for use as active ingredients of a pharmaceutical composition in the treatment of obesity and/or neurodegeneration.

8. A pharmaceutical composition comprising as an active ingredient at least one lipidized cocaine- and amphetamine-regulated transcript peptide analogue according to any one of claims 1 to 4 and one or more pharmaceutically acceptable carriers, polymers, or excipients.

9. Use of lipidized cocaine- and amphetamine-regulated transcript peptide analogue according to any one of claims 1 to 4 for the manufacturing of medicament for the treatment of obesity and/or neurodegeneration.

10. Method of treatment of obesity and/or neurodegeneration, comprising a step of administering at least one lipidized cocaine- and amphetamine-regulated transcript peptide analogue of general formula 1 according to claim 1 to a subject in need of such treatment.
